# EUROPÄISCHE PATENTSCHRIFT

(11) **EP 4 416 260 B1**
(45) Veröffentlichungstag und Bekanntmachung des Hinweises auf die Patenterteilung: **30.07.2025**
(21) Anmeldenummer: 22802566.4
(22) Anmeldetag: 12.10.2022
(51) Int. Cl.: C12M 1/26, B30B 9/12, B65G 53/08, C02F 11/121, C12M 1/00, F16C 25/02, F16C 3/02, C02F 11/125, B65G 33/24, B65G 33/14

(54) **WENDELSCHRAUBENFÖRDEREINHEIT, SCHNECKENWELLE, SCHNECKENPRESSE SOWIE VERFAHREN ZUM BEREITSTELLEN EINER SCHNECKENWELLE**
WORM SCREW CONVEYOR UNIT, SCREW SHAFT, SCREW PRESS AND METHOD FOR PROVIDING A SCREW SHAFT
UNITÉ DE TRANSPORT À VIS HÉLICOÏDALE, VIS SANS FIN, PRESSE À VIS SANS FIN, AINSI QUE PROCÉDÉ DE FOURNITURE UNE VIS SANS FIN

(30) Priorität: 15.10.2021 EP 21202998
(43) Veröffentlichungstag der Anmeldung: 21.08.2024
(73) Patentinhaber: Kanadevia Inova AG, 8005 Zürich (CH)
(72) Erfinder: EUGSTER, Marc, 8108 Dällikon (CH); SCHATZ, Adrian, 3172 Niederwangen (CH); GSPONER, Marc, 9536 Schwarzenbach (CH); GSPONER, Patrik, 9242 Oberuzwil (CH); SCHMIDT, Daniel, 9242 Oberuzwil (CH)
(74) Vertreter: Schaad, Balass, Menzl & Partner AG
(86) Internationale Anmeldenummer: PCT/EP2022/078334
(87) Internationale Veröffentlichungsnummer: WO 2023/062052

(56) Entgegenhaltungen:
- EP-A1- 0 098 340
- EP-A1- 2 792 739
- EP-A2- 1 072 574
- WO-A1-2015/189271
- CN-U- 201 835 838
- CN-U- 203 112 016
- CN-U- 212 831 025
- GB-A- 859 416
- US-A- 1 866 077
- US-A- 5 429 581
- US-A1- 2017 087 788

## Beschreibung

### Technisches Gebiet

Die vorliegende Erfindung betrifft eine Wendelschraubenfördereinheit nach dem Oberbegriff des Anspruchs 1.

Die vorliegende Erfindung betrifft des Weiteren eine Schneckenwelle, mit anderen Worten eine Förderschnecke, nach dem Oberbegriff des Anspruchs 6.

Die vorliegende Erfindung betrifft ferner eine Schneckenpresse nach dem Oberbegriff des Anspruchs 14.

Die vorliegende Erfindung betrifft schliesslich ein nach dem Oberbegriff des Anspruchs 15 ausgebildetes Verfahren zum Bereitstellen einer Schneckenwelle.

### Stand der Technik

Der allgemeine Aufbau einer Schneckenwelle zum Einsatz in einer Schneckenpresse sowie einer Schneckenpresse zum Entwässern einer Suspension ist zum Beispiel in den Druckschriften EP 1 072 574 A2, US 2017/08778 A1 und EP 2 792 739 A1 beschrieben.

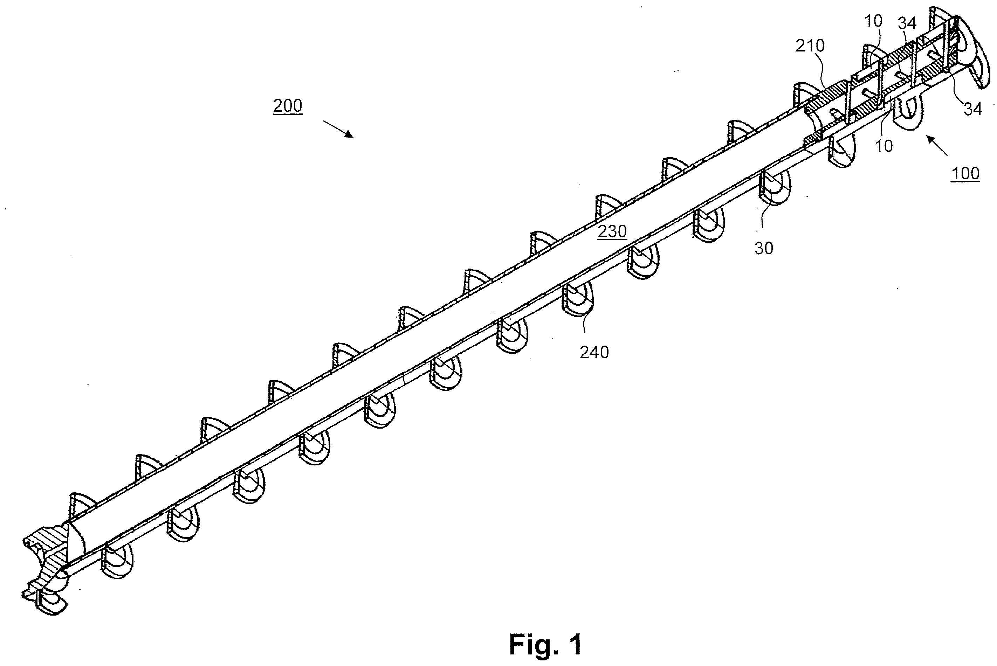

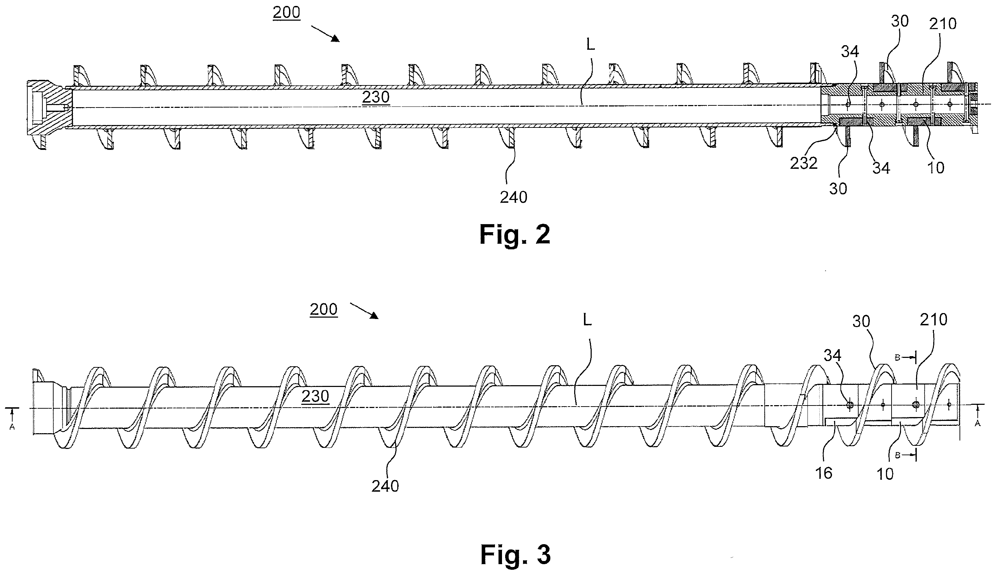

Dokument EP 1 072 574 A2 offenbart eine Vergärungsanlage mit einem Fermenter und einer Entwässerungsvorrichtung als zylindrischer Schneckenförderer. Dieser Schneckenförderer erstreckt sich entlang einer linearen Achse. Wie in den Figuren 1 bis 3 gezeigt, bilden die Wendelelemente mit der Schneckenwelle eine feste Einheit.

Die Druckschrift US 2017/08778 A1 offenbart eine Schneckenpresse zum Entwässern einer wässrigen Suspension organischen Materials. Die Vorrichtung weist eine Presskammer auf, in der sich eine Pressschnecke mit Wendelelementen schraubenartig windet und um ihre Längsachse rotierbar gelagert ist. Um eine einfache und schnelle Wartung der Schneckenpresse zu erlauben, lehrt die US 2017/08778 A1 mehrteilige hochschwenkbare Siebkörbe vorzusehen, welche eine leichte Zugänglichkeit zur Presskammer und einen Siebwechsel durch eine einzelne Person ermöglichen.

Das Dokument EP 2 792 739 A1 offenbart eine Doppelschneckenwelle in einer Presskammer, wie beispielsweise für Zellaufschlussextruder bekannt. Die Wendelschraubenfördereinheiten besitzen sogenannte Räumzinken auf den nebeneinander liegenden Schneckenwellen.

Anaerobe Vergärung (A[naerobic]D[igestion]) ist ein Bioprozess, der die energetische Verwertung organischer Reststoffe, insbesondere Grüngüter, durch Umwandlung in Biogas ermöglicht. Das Substrat wird unter anaeroben Bedingungen in einem Fermenter aufgeschlossen. Dabei wird ein die Abfallstoffe enthaltendes Fermentationsgut mit Flüssigkeit gemischt und die derart erhaltene Fermentationsgutsuspension in einem Fermenter anaerob fermentiert.

Das bei der Fermentation der Fementationsgutsuspension anfallende Digestat wird mittels einer Schneckenpresse entwässert, wobei unter Abscheidung von Presswasser ein Presskuchen mit einem gegenüber dem Digestat erhöhten Trockensubstanzanteil erhalten wird. Nachdem das Substrat den Fermenter verlässt, wird es also durch eine Schneckenpresse in feste und flüssige Gärreste getrennt.

Im Wesentlichen weist eine Schneckenpresse eine innenliegende Schneckenwelle mit schraubenartig gewundenen Wendelelementen und eine die Schneckenwelle ummantelnde Siebtrommel bzw. einen Siebzylinder auf. Das zu entwässernde Digestat wird über einen Digestateintrag in die Siebtrommel gepumpt. Die Schneckenwelle rotiert und fördert so das Digestat in Richtung eines Presskuchenaustrags, wo sich ein Widerstand, beispielsweise ein Gegendruckkonus, befindet, gegen den das Digestat gepresst wird. Das Presswasser läuft über einen Presswasserablauf ab.

Je weiter das Digestat nach vorne, also in Richtung des Widerstands transportiert wird, desto größer ist die Volumenreduktion und desto höher ist der Druck auf das Digestat. Dadurch wird noch mehr Flüssigkeit aus dem Digestat gepresst, die als Presswasser abläuft. Der entwässerte Presskuchen verlässt die Schneckenpresse über einen Presskuchenaustrag.

Beim Betrieb der Schneckenpresse werden die Wendelelemente mit der Zeit abgenutzt. Diese Abnutzung erfolgt umso schneller je mehr Druck von den Wendelementen auf das Digestat aufgebracht wird und ist im vorderen, dem Presskuchenaustrag nahen Bereich der Schneckenwelle besonders stark. Durch Abnutzung wird der Presspunkt nach hinten verlegt, wodurch das Digestat weniger stark entwässert wird. Abgenutzte Wendelemente müssen somit gewartet oder ausgetauscht werden.

Wie in den Figuren 2 und 3 der US 2017/08778 A1 gezeigt, sind die Wendelelemente üblicherweise fest mit dem Schaft der Schneckenwelle verbunden, etwa verschweisst. Bei Verschleiss einzelner Wendelemente müssen also umfangreiche Schweissarbeiten durchgeführt werden oder es muss die gesamte Schneckenwelle ersetzt werden.

Aus der Druckschrift EP 0098 340 A1 ist eine Schneckenpumpe zum Fördern von festem und körnigem Material bekannt. Um die sich besonders stark abnutzenden Endbereiche der Schneckenwendel austauschbar anzuordnen, sind im Endbereich austauschbare Hülsen vorgesehen, welche axial von der Schneckenwelle abgezogen werden können. Zum Auswechseln der Hülsen muss die Schneckenwelle aus der Schneckenpumpe ausgebaut werden.

Des Weiteren offenbart die Druckschrift GB 859 416 A einen Schneckenwendelförderer mit als Hülse aufsteckbaren Austauschwendelelementen. Um den Ausbau einzelner Abschnitte der Schneckenwelle für Wartungs- und Reparaturarbeiten zu ermöglichen, ist die Schneckenwelle aus mehreren zusammenpassenden hülsenartigen Längsabschnitten aufgebaut. Diese Längsabschnitte sind mittels halbkreisförmiger schellenartiger Lager am Schaft der Schneckenwelle lösbar befestigt. Nach dem Lösen der Lager können die hülsenartigen Längsabschnitte in axialer Richtung vom Schaft der Schneckenwelle abgezogen werden, wofür die Schneckenwelle zumindest an einer Seite aus ihrem Lager ausgebaut werden muss.

Um Schaufeln eines Schneckenförderers nach ihrem Verschleiss nicht aufwändig durch Schweissarbeiten entfernen und reparieren zu müssen, lehrt die Druckschrift CN 212 831 025 U die Schaufeln mittels Nut und Schrauben an der Welle zu befestigen. Die Schaufeln sind jeweils separate paddelartige Elemente, welche unabhängig voneinander an die Welle geschraubt sind.

### Darstellung der vorliegenden Erfindung: Aufgabe, Lösung, Vorteile

Der Erfindung liegt die Aufgabe zugrunde, eine Wendelschraubenfördereinheit der eingangs genannten Art sowie eine Schneckenwelle der eingangs genannten Art sowie eine Schneckenpresse der eingangs genannten Art sowie ein Verfahren der eingangs genannten Art derart weiterzuentwickeln, dass die Wendelschraubenfördereinheit mit geringem Wartungsaufwand gewartet werden kann. Insbesondere soll die als Austauschelement für eine Wendel ausgebildete Wendelschraubenfördereinheit austauschbar sein, ohne dass hierfür die Schneckenwelle aus deren Lager ausgebaut werden muss. Zudem sollen die Wartungskosten der Schneckenwelle gesenkt werden.

Diese Aufgabe wird durch eine Wendelschraubenfördereinheit mit den im Anspruch 1 angegebenen Merkmalen, durch eine Schneckenwelle mit den im Anspruch 6 angegebenen Merkmalen, durch eine Schneckenpresse mit den im Anspruch 14 angegebenen Merkmalen sowie durch ein Verfahren mit den im Anspruch 15 angegebenen Merkmalen gelöst. Vorteilhafte Ausgestaltungen und zweckmäßige Weiterbildungen der vorliegenden Erfindung sind in den jeweiligen abhängigen Ansprüchen gekennzeichnet.

Mithin basiert die Erfindung darauf, dass die Wendelschraubenfördereinheit radial austauschbar an einem Befestigungsbereich des Schafts der Schneckenwelle montierbar ist. So kann die Wendelschraubenfördereinheit als austauschbares Ersatzteil an einer drehbar gelagerten Schneckenwelle einer Schneckenpresse montiert werden. Die vorliegende Erfindung ermöglicht somit einen schnellen Wechsel der Wendelschraubenfördereinheit, weil aufgrund der radialen Öffnung des zur Aufnahme des Schafts ausgebildeten Grundkörpers der Wendelschraubenfördereinheit diese radial austauschbar am Schaft montierbar ist und gewechselt werden kann auch wenn der Schaft an seinen axialen Enden in mindestens einem Drehlager gelagert ist.

Mit Radial wird vorliegend eine Richtung bezeichnet, die senkrecht zur Längsachse (L) des Schneckenwellenschafts bzw. des Grundkörpers der Schneckenwelle angeordnet ist. Die radiale Austauschbarkeit ermöglicht somit einen effizienten Wechsel der Wendelemente ohne Ausbau der Schneckenwelle aus deren Lager und lange Betriebszeiten der Schneckenwelle.

Der Schaft der Schneckenwelle ist ein stabförmiges Element zur Übertragung von Drehbewegungen. Die Wendelschraubenfördereinheit kann als lösbares Ersatzteil am Befestigungsbereich des Schafts montiert werden. Die Wendelschraubenfördereinheit ist also vorteilhafterweise dazu ausgebildet, an einem Befestigungsbereich einer vorstehend dargelegten Schneckenwelle lösbar montiert zu werden. Ferner ist die Schneckenwelle vorteilhafterweise dazu geeignet in einer Schneckenpresse der vorstehend dargelegten Art montiert zu werden.

Zum lösbaren Befestigen des Grundkörpers des Wendelelements am Befestigungsbereich weist die Wendelschraubenfördereinheit mindestens ein Befestigungselement auf. Vorteilhafterweise ist also das Befestigungselement der Wendelschraubenfördereinheiten der vorstehend dargelegten Art lösbar mit dem Befestigungsmittel, insbesondere mit der Befestigungsstruktur, der Schneckenwelle der vorstehend dargelegten Art verbindbar.

Bei einem vorteilhaften Ausführungsbeispiel ist der Grundkörper ein Fusselement des Wendelelements, das am radial inneren Bereich des Wendelelements angeordnet ist. Dabei ist das Befestigungselement dazu ausgebildet, das Fusselement am Befestigungsbereich lösbar zu befestigen. Dabei ist das Fusselelement nach Art eines Fusses auf den Befestigungsbereich aufsetzbar und das Wendelelement erstreckt sich vom Fusselement nach aussen weg.

Um das Wendelelement auf einfache Weise radial lösbar an der Schneckenwelle zu befestigen, kann das Befestigungselement sich in seiner Gebrauchsstellung radial in einen zum Stützen der Wendelschraubenfördereinheit ausgebildeten Stützbereich des Schafts hinein erstrecken und das auf dem Befestigungsbereich aufliegende Fusselement am Stützbereich befestigen.

Bei einem vorteilhaften Ausführungsbeispiel weist der Stützbereich des Schafts mindestens zwei Wendelelemente auf, wobei jedem Wendelelement mindestens ein Befestigungselement zugeordnet ist und die Befestigungselemente der zueinander benachbarten Wendelelemente sowohl axial als auch radial versetzt zueinander angeordnet sind. Das Befestigungselement ist vorzugsweise eine Zylinderschraube und das Befestigungsmittel ein Gewinde. Alternativ kann das Befestigungselement mit dem Befestigungsmittel auch nach Art eines lösbaren Schnappverschlusses zusammenwirken.

Bevorzugt ist die Innenfläche des Grundkörpers der Wendelschraubenfördereinheit der vorstehend dargelegten Art dazu ausgebildet, auf dem Befestigungsbereich der Schneckenwelle der vorstehend dargelegten Art aufzuliegen, insbesondere zu lagern. Mit anderen Worten ist der Befestigungsbereich einem Bereich der Schneckenwelle zugeordnet, der zum Stützen des Grundkörpers der Wendelschraubenfördereinheit ausgebildet ist.

Um ein Auswechseln der Wendelschraubenfördereinheit an der Schneckenwelle besonders einfach zu gestalten, ist die Innenfläche des Grundkörpers vorteilhafterweise derart geformt, dass durch Aufliegen der Innenfläche des Grundkörpers auf dem Befestigungsbereich der Schneckenwelle die Ausrichtung des Wendelelements der Wendelschraubenfördereinheit an der Schneckenwelle definiert und eine radiale Bewegbarkeit des Grundkörpers auf der Schneckenwelle zumindest eingeschränkt ist. Somit weisen vorteilhafterweise die Innenfläche des Grundkörpers sowie der Befestigungsbereich eine Form oder Struktur auf, welche zusammenwirkt und ein radiales Verrutschen oder eine Drehbewegung der Wendelschraubenfördereinheit um den Schaft verhindert. Dabei wird eine radiale Drehbewegung der Wendelschraubenfördereinheit um den Schaft bereits durch das Zusammenwirken der Form oder Struktur des Befestigungsbereichs mit der Form oder Struktur der Innenfläche des Grundkörpers verhindert. Das Befestigungsmittel dient dann zur weiteren oder zusätzlichen Fixierung. Wie unten näher beschrieben, kann diese mittels Form oder Struktur erzielte Befestigung beispielsweise mittels einer vierkantartigen Form des Befestigungsbereichs erreicht werden. Der Befestigungsbereich ist zum lösbaren Befestigen des Befestigungselements der Wendelschraubenfördereinheit ausgebildet und einem zum Stützen der Wendelschraubenfördereinheit ausgebildeten Stützbereich des Schafts der Schneckenwelle zugeordnet. Der Befestigungsbereich hat mindestens ein Befestigungsmittel, etwa eine Befestigungsstruktur. Vorteilhafterweise weist der Stützbereich mindestens zwei Befestigungsbereiche auf, so dass vorteilhafterweise mindestens zwei Wendelschraubenfördereinheiten der vorstehend dargelegten Art am Stützbereich der Schneckenwelle lösbar befestigbar sind.

Vorteilhafterweise weist die Schneckenwelle also mindestens zwei Wendelschraubenfördereinheiten auf. Dabei sind die Befestigungsbereiche des Stützbereichs vorzugsweise derart ausgebildet, dass benachbarte Wendelschraubenfördereinheiten jeweils axial sowie radial versetzt, beispielsweise versetzt spiegelbildlich, zueinander angeordnet sind. Bei diesem vorteilhaften Ausführungsbeispiel weist die Schneckenwelle mindestens zwei axial sowie radial versetzt zueinander angeordnete Wendelelemente auf. Dementsprechend weist der Stützbereich mindestens zwei axial sowie radial versetzt zueinander angeordnete Befestigungsbereiche auf. Vorteilhafterweise sind bei diesem Ausführungsbeispiel auch die Befestigungselemente der benachbarten Wendelschraubenfördereinheiten axial sowie radial zueinander versetzt angeordnet, beispielsweise um 90 Grad versetzt, etwa senkrecht zueinander, angeordnet.

Die Wendelelemente weisen eine teilweise Wendelumdrehung, beispielsweise eine viertel bis eine halbe Wendelumdrehung auf. Mehrere benachbarte Wendelelemente ergeben gemeinsam eine spiralförmige oder helixförmige oder schraubenartige Wendelumdrehung. Mehrere Wendelumdrehungen ergeben gemeinsam die schraubenartig oder helixartig oder spiralartig gewundene Wendel der Schneckenwelle. Da bei der vorliegenden Erfindung die Wendelelemente austauschbar sind, kann im Vergleich zu einer aus dem Stand der Technik bekannten Schneckenwelle mit unlösbaren Wendelelementen ein erhöhter Materialabtrieb dieser Wendelelemente in Kauf genommen werden und der Widerstand der Schneckenpresse erhöht, beispielsweise der Gegendruckkonus vergrössert oder verlängert werden, um einen grösseren Kraftaufwand zum Entwässern des Digestats bereitzustellen.

Der Grundkörper der Wendelschraubenfördereinheit weist eine radiale Öffnung auf. Durch diese Öffnung kann der Befestigungsbereich in radialer Richtung, also in einer senkrecht zur Längsachse (L) des Grundkörper angeordneten Richtung, durchgeführt und an der Innenfläche des Grundkörpers angeordnet werden. Mit anderen Worten ist der Stützbereich durch die Öffnung hindurchführbar und in einem vom konkaven Grundkörper umgebenen Hohlraum aufnehmbar.

Das Befestigungselement ist bevorzugt zum Bereitstellen einer formschlüssigen Verbindung zwischen dem Grundkörper und dem Stützbereich oder Wellenkörperelement des Schafts ausgebildet, beispielsweise eine Schraube, etwa eine Zylinderschraube. Alternativ könnte das Befestigungselement auch dazu ausgebildet sein eine Schnappverbindung oder eine Klemmverbindung bereitzustellen.

Um den Grundkörper auf dem Stützbereich gestützt anordnen zu können, ist die Innenfläche des Grundkörpers komplementär zum Befestigungsbereich ausgebildet und die Aussenseite des Grundkörpers ist konkav gebogen und verläuft, bevorzugt halbschalenförmig oder halbkreisförmig, um die Längsachse.

Der Befestigungsbereich weist mindestens eine zum Stützen des Grundkörpers, insbesondere der Innenfläche des Grundkörpers, ausgebildete Auflagefläche auf. Um ein Verrutschen des Grundkörpers auf der Auflagefläche zu verhindern, weist die Innenfläche der Mantelfläche des Grundkörpers vorteilhafterweise mindestens eine zum ortsfesten Anordnen des Grundkörpers am Befestigungsbereich ausgebildete Kontur auf und ist vorteilhafterweise komplementär zum Befestigungsbereich bzw. zur Auflagefläche des Befestigungsbereichs ausgebildet. Bevorzugt ist die Kontur der Innenfläche des Grundkörpers der Wendelschraubenfördereinheit im Querschnitt U-förmig oder n-förmig ausgebildet.

Besonders schnell und einfach kann die Wendelschraubenfördereinheit an der Schneckenwelle ausgetauscht werden, wenn das Wendelelement derart geformt ist, dass durch Aufliegen der Innenfläche des Grundkörpers auf dem Befestigungsbereich der Schneckenwelle das Wendelelement derart ausgerichtet wird, dass es den Verlauf der schraubenartig oder spiralartig gewundenen Wendel der Schneckenwelle zumindest im Wesentlichen fortsetzt.

Um ein radiales Verrutschen des Grundkörpers auf dem Schaft zu vermeiden, kann die Innenfläche der Mantelfläche beispielsweise rechtwinklig geformt, gebogen, gekrümmt oder stufenartig geformt sein. Beispielsweise kann der Befestigungsbereich im Querschnitt u-förmig und die Innenfläche des Grundkörpers im Querschnitt n-förmig ausgebildet sein, oder umgekehrt.

Der Stützbereich des Schafts kann zumindest im Bereich des Befestigungsbereichs als Vierkant ausgebildet sein. Alternativ kann der Befestigungsbereich durch mindestens eine Ausnehmung oder mindestens eine Nut des ansonsten zylinderförmigen Stützbereichs gebildet sein. Beispielsweise kann der Befestigungsbereich eine u-förmige oder n-förmige Ausnehmung des ansonsten zylinderförmigen Stützbereichs sein. Um ein Verrutschen der Wendelschraubenfördereinheiten auf dem Befestigungsbereich zu verhindern, hat dieser vorteilhafterweise einen Vierkantsitz.

Das Wendelelement weist einen Teil oder einen Abschnitt einer Wendelumdrehung, bevorzugt eine halbe Wendelumdrehung auf. Alternativ kann es auch beispielsweise eine drittel Wendelumdrehung oder eine viertel Wendelumdrehung aufweisen. Die Wendelschraubenfördereinheit weist mindestens eine Wendeleinheit, bevorzugt mehrere, beispielsweise vier nebeneinander angeordnete Wendeleinheiten auf. Zur passgenauen Verbindung können Wendelelemente nebeneinander angeordneter Wendelschraubenfördereinheit miteinander verschweisst und geschliffen werden.

Vorteilhafterweise erstreckt sich das Befestigungselement in seiner Gebrauchsstellung radial durch den Stützbereich und ragt in den Grundkörper oder durch den Grundkörper mindestens einer am Stützbereich angeordneten Wendelschraubenfördereinheit. Besonders bevorzugt ragt das Befestigungselement an seinem aus dem Stützbereich austretenden Ende in den Grundkörper einer Wendelschraubenfördereinheit. So kann das Befestigungselement zum Austausch der Wendelschraubenfördereinheit nur teilweise gelöst werden, beispielsweise nur vom Grundkörper der Wendelschraubenfördereinheit gelöst werden, aber noch am Stützbereich befestigt bleiben. Dies verhindert ein Verlieren des Befestigungselements.

Die Schneckenwelle weist vorteilhafterweise mindestens ein Wendelschraubenförderelement gemäss der vorstehend dargelegten Art auf, wobei das Wendelschraubenförderelement lösbar am Befestigungsbereich befestigbar ist.

Neben dem Wendelschraubenförderelement kann der Schaft des Weiteren einen sich entlang der Längsachse erstreckenden zylinderförmigen Wellenkörper mit mindestens einer schraubenartig um den Wellenkörper gewundenen Wellenkörperwendel aufweisen. Der Befestigungsbereich des sich ebenfalls entlang der Längsachse erstreckenden Stützbereichs weist einen im Vergleich zum zylinderförmigen Wellenkörper geringeren Querschnittsdurchmesser auf. Dies ermöglicht es die Wendelschraubenfördereinheit derart am Stützbereich anzuordnen, dass die Oberseite ihrer Mantelfläche bündig mit dem zylinderförmigen Wellenkörper abschliesst.

Vorteilhafterweise ist der Stützbereich exzentrisch, insbesondere an einem endnahen, beispielsweise an einem endständigen, Bereich des zylinderförmigen Wellenkörpers angeordnet. So kann die Schneckenwelle derart in einer Schneckenpresse angeordnet werden, dass das mindestens eine austauschbare Wendelelement im vorderen, dem Presskuchenaustrag nahen Bereich der Schneckenpresse angeordnet ist. Der Austausch nur der am stärksten durch Abnutzung betroffenen Frontwendel ermöglicht die Schneckenwelle einfach, schnell und mit geringem Kostenaufwand zu warten.

Bei einer vorteilhaften Ausführungsform des Verfahrens der vorliegenden Erfindung wird die Schneckenwelle gewartet, indem
- mindestens ein Befestigungsmittel, das eine erste Wendelschraubenfördereinheit an dem Befestigungsbereich befestigt, gelöst wird,
- die erste Wendelschraubenfördereinheit von der Schneckenwelle entnommen wird,
- eine weitere Wendelschraubenfördereinheit am Befestigungsbereich angeordnet wird und
- mittels einem dieser weiteren Wendelschraubenfördereinheit zugeordneten Befestigungsmittel lösbar an dem Befestigungsbereich befestigt wird.

Die vorliegende Erfindung betrifft schliesslich die Verwendung mindestens einer Schneckenwelle der vorstehend dargelegten Art in einer Schneckenpresse der vorstehend dargelegten Art zur Trennung von festen und flüssigen Gärresten von mittels eines Fermenters behandelten biogenen Reststoffen, insbesondere Grüngütern.

### Kurze Beschreibung der Zeichnungen

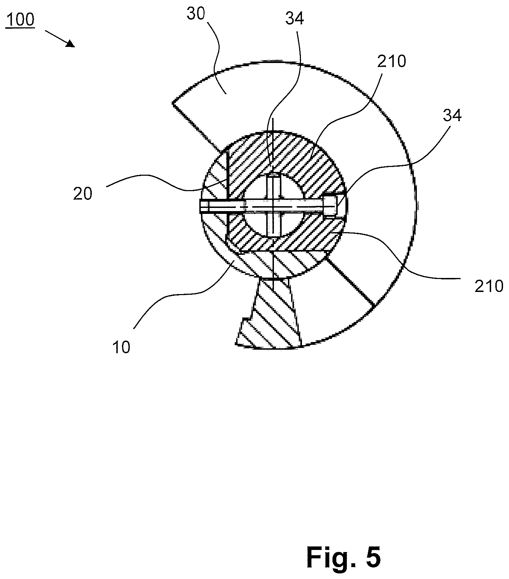

Wie bereits vorstehend erörtert, gibt es verschiedene Möglichkeiten, die Lehre der vorliegenden Erfindung in vorteilhafter Weise auszugestalten und weiterzubilden. Hierzu wird einerseits auf die dem Anspruch 1 sowie dem Anspruch 6 nachgeordneten Ansprüche verwiesen, andererseits werden weitere Ausgestaltungen, Merkmale und Vorteile der vorliegenden Erfindung nachstehend unter anderem anhand des durch die Figuren 1 bis 7 veranschaulichten Ausführungsbeispiels näher erläutert.

Es zeigt:
- Fig. 1: in perspektivischer Darstellung einen Längsschnitt eines ersten Ausführungsbeispiels für eine Schneckenwelle gemäss der vorliegenden Erfindung, mit einer lösbar daran anordbaren Wendelschraubenfördereinheit gemäss der vorliegenden Erfindung, die nach dem Verfahren der vorliegenden Erfindung austauschbar ist;
- Fig. 2: in Längsschnittdarstellung die Schneckenwelle aus Figur 1;
- Fig. 3: eine Seitenansicht der Schneckenwelle aus Figur 1;
- Fig. 4: eine Detailansicht des vorderen Bereichs der Schneckenwelle aus Figur 2;
- Fig. 5: einen Querschnitt der Wendelschraubenfördereinheit aus Figur 1 entlang der in Figur 3 gezeigten Linie B-B;
- Fig. 6: in Explosionsdarstellung die Schneckenwelle aus Figur 1; und
- Fig. 7: ein Ausführungsbeispiel für eine Schneckenpresse gemäss der vorliegenden Erfindung mit der Schneckenwelle aus Figur 1.

Gleiche oder ähnliche Ausgestaltungen, Elemente oder Merkmale sind in den Fig. 1 bis 7 mit identischen Bezugszeichen versehen.

### Bester Weg zur Ausführung der Erfindung

Figur 1 zeigt ein Ausführungsbeispiel einer Wendelschraubenfördereinheit 100 für eine Schneckenwelle 200. Die Wendelschraubenfördereinheit 100 weist einen sich entlang einer Längsachse L erstreckenden Grundkörper 10 auf. Der Grundkörper weist zwei Endbereiche auf, welche sich senkrecht zur Längsachse erstrecken, mit anderen Worten in Richtung der Längsachse einander gegenüberliegen. Die Endbereiche 12, 14 sind durch eine Mantelfläche miteinander verbunden, wobei um eine Oberseite 16 oder Aussenfläche der Mantelfläche mindestens ein schraubenartig gewundenes Wendelelement 30 angeordnet ist.

Bei dem in den Figuren 1 bis 3 gezeigten Ausführungsbeispiel weist die Schneckenwelle 200 mehrere, beispielsweise zwei, Wendelelemente 30 auf. Zur passgenauen Verbindung der Wendelelemente 30 können diese miteinander verschweisst und geschliffen werden.

Das mindestens eine Wendelelement 30 weist eine teilweise, insbesondere eine viertel bis eine halbe, Wendelumdrehung auf. Der Grundkörper ist konkav gebogen, also nach innen gewölbt, und weist eine radiale Öffnung 20 auf. Mit anderen Worten ist der Grundkörper hohl ausgebildet und verläuft bogenförmig um die Längsachse L. Bevorzugt verläuft der Grundkörper teilkreisförmig, beispielsweise halbschalenartig, um die Längsachse L.

Die Wendelschraubenfördereinheit 100 ist zum austauschbaren Einbau in eine Schneckenwelle 200, bevorzugt in eine Schneckenwelle 200 einer zum Entwässern von mittels anaerober Vergärung von organischem Material erhaltenem Digestat ausgebildeten Schneckenpresse 300, ausgebildet.

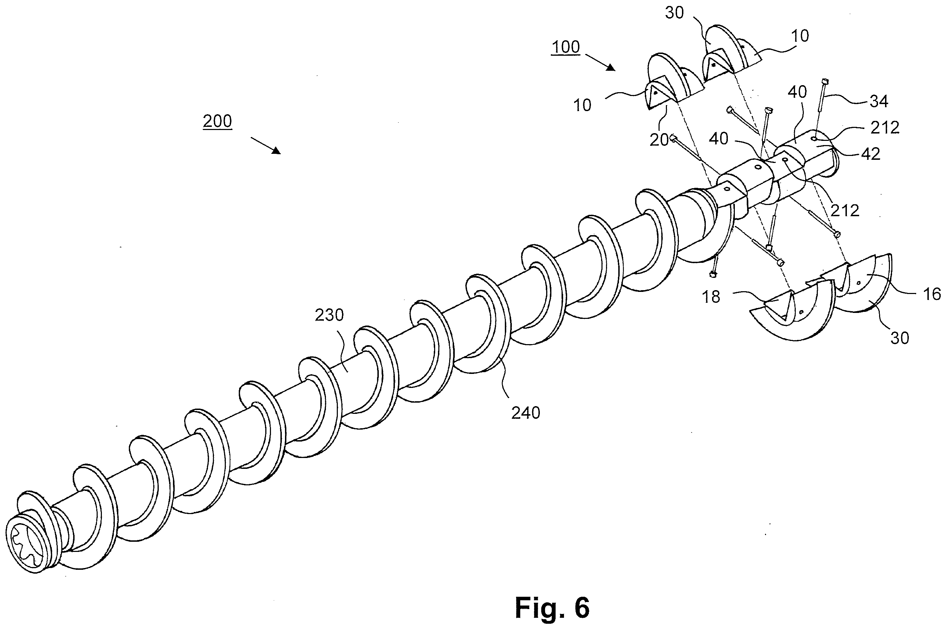

Figur 6 zeigt eine Explosionsdarstellung der Schneckenwelle 200. Diese weist vier Wendelschraubenfördereinheiten 100 auf, welche zusammen zwei umlaufende Wendel bilden. Jeweils benachbarte Wendelschraubenfördereinheiten 100 sind radial um 180 Grad versetzt, also spiegelbildlich, zueinander angeordnet. Zum lösbaren Befestigen des Grundkörpers 100 an den Befestigungsbereichen 40 des Stützbereichs 210 weisen die Wendelschraubenfördereinheit jeweils zwei Zylinderschrauben 34 auf. Die Befestigungsbereiche 40 sind nach Art eines teilweisen Vierkants bzw. rechtwinklig geformt. Die den Wendelschraubenfördereinheiten 100 bzw. den Befestigungsbereichen 40 gegenüberliegenden Bereiche des Stützbereichs 210 sind als Füllkörper ausgebildet. Diese schützen und stabilisieren den Stützbereich 210. Diese Füllkörper können fest mit dem Stützbereich 210 verbunden, beispielsweise einstückig mit dem Stützbereich 210 ausgebildet, oder lösbar am Stützbereich 210 befestigt sein. Die Füllkörper sind konkav um die Längsachse L gebogen.

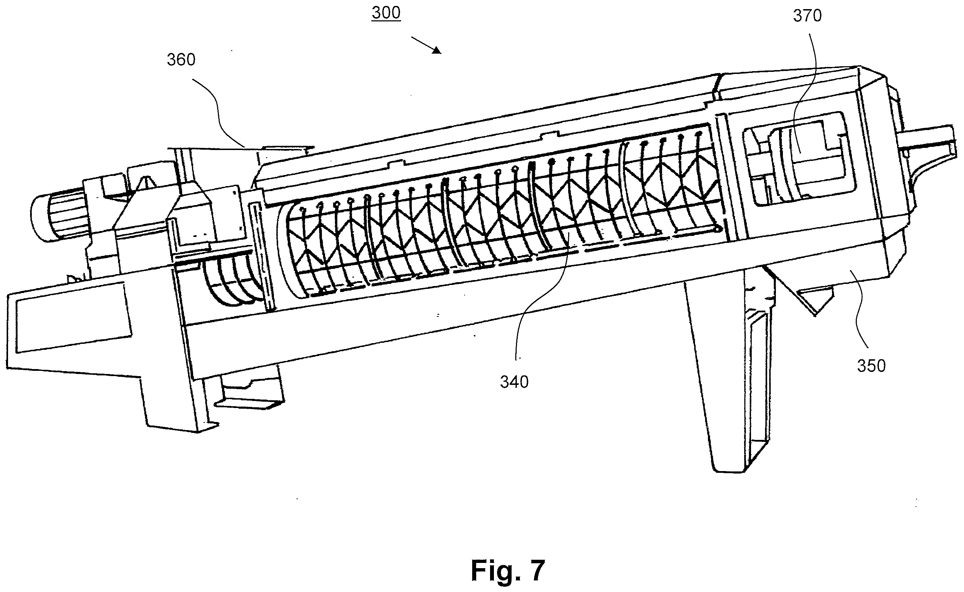

Wie in Figur 7 gezeigt, weist die Schneckenpresse 300 eine innenliegende Schneckenwelle 200 mit sich nach aussen weg erstreckenden schraubenartig gewundenen Wendelelementen 30, 210 und eine die Schneckenwelle 200 ummantelnde Siebtrommel 340 auf. Die Siebtrommel 340 kann schwenkbare Siebschalen aufweisen, was einen einfachen Zugang zur Schneckenwelle und einen schnellen Wechsel von Sieb- und Verschleissteilen ermöglicht.

Zum austauschbaren Einbau in eine Schneckenwelle 200 weist die Wendelschraubenfördereinheit 100
- an der Mantelfläche des Grundkörpers 10 eine zur Aufnahme eines Stützbereichs 210 eines Schafts 230 ausgebildete Innenfläche 18 auf, und
- mindestens ein zum lösbaren Befestigen der Innenfläche 18 am Stützbereich 210 ausgebildetes Befestigungselement 34 auf.

Die Wendelschraubenfördereinheit 100 ist also ein lösbar befestigbares oder austauschbares Element für eine Schneckenwelle 200.

Bei einem, vorliegend nicht gezeigten Ausführungsbeispiel der vorliegenden Erfindung kann die Schneckenwelle 200 ausschliesslich austauschbare Wendelelemente 30 aufweisen. Alternativ ist es, wie in den Figuren 1 bis 7 gezeigt, möglich nur einen Teilbereich, beispielsweise einen vorderen, in Gebrauchsstellung einem Presskuchenaustrag 350 nahen Bereich, der Schneckenwelle 200 als austauschbare Wendelschraubenfördereinheit 100 auszubilden.

Die in den Figuren 1 bis 7 gezeigte Schneckenwelle weist neben der austauschbaren Wendelschraubenfördereinheit 100 des Weiteren einen Schaft 230 mit einem zylinderförmigen Wellenkörper und mit einer schraubenartig um den Schaft 230 gewundenen Wellenkörperwendel 240 auf. Im Bereich des zylinderförmigen Wellenkörpers ist die Wellenkörperwendel 240 unlösbar mit dem Schaft 230 verbunden, beispielsweise einstückig mit dem Schaft 230 ausgebildet oder mit dem Schaft 230 verschweisst.

Der Stützbereich 210 der Wendelschraubenfördereinheit 100 kann unlösbar, beispielsweise mittels der Technik des Schweissens, etwa mittels einer Wurzelnaht 232, mit dem zylinderförmigen Wellenkörper verbunden sein. Alternativ kann der Stützbereich 210 auch einstückig mit dem zylinderförmigen Wellenkörper des Schafts 230 ausgebildet sein.

Die Wendelschraubenfördereinheiten 100 sind derart angeordnet, dass die Wendelelemente 30 den Verlauf der Wellenkörperwendel 240 des zylinderförmigen Wellenkörpers fortsetzen und mit diesen eine gemeinsame Wellenkörperwendeleinheit bilden.

Der Grundkörper 10 der Wendelschraubenfördereinheit 100 ist konkav gebogen.

### BERICHTIGTES BLATT (REGEL 91) ISA/EP

Dabei kann der Grundkörper derart ausgebildet sein, dass in Gebrauchsstellung der Schneckenwelle 200 der Stützbereich 210 vollständig von den Grundkörpern der am Stützbereich 210 angeordneten Wendelschraubenfördereinheiten 100 ummantelt ist (nicht gezeigt). Beispielsweise können jeweils zwei als Halbschalen ausgebildete Wendelschraubenfördereinheiten 100 das Wellenkörperelement 210 vollständig ummanteln.

Alternativ kann der Grundkörper 10 ein Fusselement 32 des Wendelelements 30 sein, das am radial inneren Bereich des Wendelelements 30 angeordnet ist, und in einem als Nut ausgebildeten Befestigungsbereich 40 des Stützbereichs 210 austauschbar angeordnet werden kann.

Bei dem in den Figuren 1 bis 7 gezeigten Ausführungsbeispiel ist die Wendelfrequenz der Wendelelemente 30 identisch mit der Wendelfrequenz der Wellenkörperwendel 240. Die Wendelfrequenz, bzw. die Steigung der Wendelelemente 30, könnte aber auch im Vergleich zur Wendelfrequenz der Wellenkörperwendel 240 zunehmen.

Zum drehbaren Lagern der Schneckenwelle 200 in der Schneckenpresse 300 weist diese mindestens ein Drehlager 250 auf. Zur Entlastung des Getriebes der Schneckenpresse 300 von radialen und axialen Kräften kann die Schneckenwelle 200 doppelt gelagert sein. Dies ermöglicht längere Standzeiten der Schneckenpresse 300.

Ein bevorzugtes Material für die Wendelschraubenfördereinheit 100 der vorstehend dargelegten Art sowie für die Schneckenwelle 200 der vorstehend dargelegten Art ist Stahl.

### Bezugszeichenliste

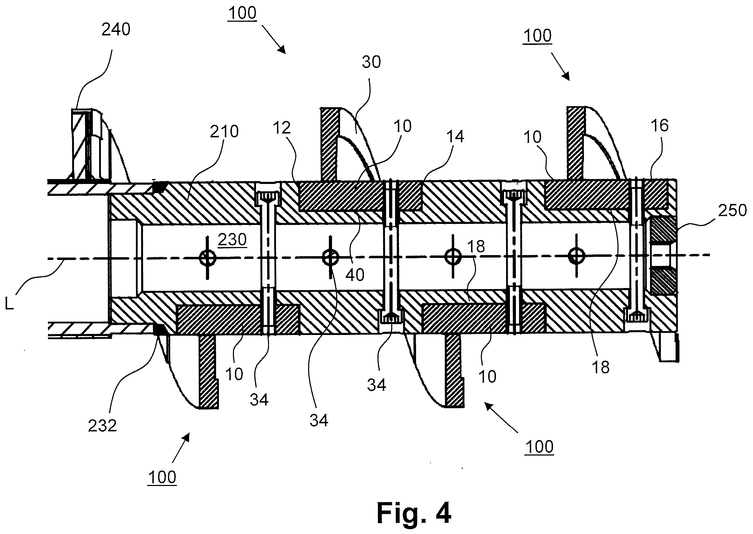

- 10: Grundkörper, insbesondere Fusselement des Wendelelements 30
- 12: erster Endbereich des Grundkörpers, insbesondere erster sich senkrecht zur Längsachse erstreckender Endbereich des Grundkörpers
- 14: zweiter Endbereich des Grundkörpers, insbesondere zweiter sich senkrecht zur Längsachse erstreckender Endbereich des Grundkörpers
- 16: Oberseite, insbesondere Aussenseite, der Mantelfläche des Grundkörpers 10
- 18: Innenseite der Mantelfläche des Grundkörpers 10
- 20: radiale bzw. konkave Öffnung des Grundkörpers 10
- 30: Wendelelement der Wendelschraubenfördereinheit 100
- 34: Befestigungselement des Wendelelements 30
- 40: Befestigungsbereich, insbesondere U-förmig ausgebildeter Befestigungsbereich, Vierkant oder Aufnahmenut, des Grundkörpers 10
- 42: Füllkörper
- 100: Wendelschraubenfördereinheit
- 200: Schneckenwelle oder Förderschnecke oder Pressschnecke, insbesondere stabförmiger Schneckenwellenschaft mit schraubenartig oder spiralartig gewundenen Wendelelementen 240, 30
- 210: Wellenkörperelement oder zum Stützen des Grundkörpers 10 der Wendelschraubenfördereinheit 100 ausgebildeter Stützbereich des Schneckenwellenschafts 230, insbesondere Schaftauflagebereich oder Stützbereich des Schafts 230 der Schneckenwelle 200
- 212: Befestigungsmittel, insbesondere Gewinde, des Wellenkörperelements 210
- 230: Schaft oder stabförmiger Grundkörper der Schneckenwelle 200, insbesondere mit zylinderförmigem Wellenkörper und zum Stützen des Grundkörpers 10 der Wendelschraubenfördereinheit 100 ausgebildetem Stützbereich 210
- 232: Schweissnaht, insbesondere Wurzelnaht, zum Verbinden des zylinderförmigen Wellenkörpers 230 mit dem Grundkörper 10
- 240: spiralförmig oder helixförmig oder schraubenartig gewundene Wellenkörperwendel oder Wendelelement der Schneckenwelle 200
- 250: Drehlager (vgl. Figur 4)
- 300: Schneckenpresse
- 340: Siebtrommel
- 350: Presskuchenaustrag
- 360: Digestateintrag
- 370: Widerstand, insbesondere Gegendruckkonus
- L: Längsachse

## Patentansprüche

1. Wendelschraubenfördereinheit (100) ausgebildet als Austauschelement für eine schraubenartig oder spiralartig gewundene Wendel (240) einer Schneckenwelle (200), aufweisend einen sich entlang einer Längsachse (L) erstreckenden Grundkörper (10), wobei der Grundkörper (10) zwei axiale Endbereiche (12, 14) sowie eine die Endbereiche (12, 14) miteinander verbindende Mantelfläche aufweist, wobei an einer Oberseite (16) der Mantelfläche mindestens ein sich von der Oberseite (16) radial nach aussen weg erstreckendes, schraubenartig gewundenes Wendelelement (30) angeordnet ist,
**dadurch gekennzeichnet, dass**
- die Oberseite (16) der Mantelfläche schalenartig konkav um die Längsachse (L) gebogen ist und der Grundkörper (10) eine radiale Öffnung (20) aufweist, wobei die radiale Öffnung (20) derart ausgebildet ist, dass ein Befestigungsbereich (40) eines Schafts (230) der Schneckenwelle (200) in senkrecht zur Längsachse (L) ausgebildeter Richtung durch die Öffnung (20) hindurchführbar und in einem durch den Grundkörper (10) geformten Hohlraum anordbar ist,
- das Wendelelement (30) höchstens eine halbe Wendelumdrehung aufweist,
- die Mantelfläche des Grundkörpers (10) eine zum Aufliegen auf dem Befestigungsbereich (40) ausgebildete Innenfläche (18) aufweist, und
- die Wendelschraubenfördereinheit (100) mindestens ein zum lösbaren Befestigen des Grundkörpers (10) am Befestigungsbereich (40) ausgebildetes Befestigungselement (34) aufweist.

2. Wendelschraubenfördereinheit nach Anspruch 1, **dadurch gekennzeichnet, dass** der Grundkörper (10) halbkreisförmig oder halbschalenförmig um die Längsachse (L) verläuft, insbesondere als Halbschale ausgebildet ist, und das Wendelelement (30) eine halbe Wendelumdrehung aufweist.

3. Wendelschraubenfördereinheit nach Anspruch 1 oder 2, **dadurch gekennzeichnet, dass** die Innenfläche (18) des Grundkörpers (10) derart geformt ist, dass durch Aufliegen der Innenfläche (18) des Grundkörpers (10) auf dem Befestigungsbereich (40) der Schneckenwelle (200) die Ausrichtung des Wendelelements (30) der Wendelschraubenfördereinheit (100) an der Schneckenwelle (200) definiert und eine radiale Bewegbarkeit des Grundkörpers (10) auf der Schneckenwelle (200) zumindest einschränkt ist.

4. Wendelschraubenfördereinheit nach Anspruch 3, **dadurch gekennzeichnet, dass** zum Ausrichten des Wendelelements (30) an der Schneckenwelle (200) die Innenfläche (18) der Mantelfläche eine komplementär zum Befestigungsbereich (40) des Schafts (230) geformte Kontur aufweist.

5. Wendelschraubenfördereinheit nach Anspruch 2 oder 3, **dadurch gekennzeichnet, dass** das Wendelelement (30) derart geformt ist, dass durch Aufliegen der Innenfläche (18) des Grundkörpers (10) auf dem Befestigungsbereich (40) der Schneckenwelle (200) das Wendelelement (30) zumindest im Wesentlichen derart ausgerichtet wird, dass es den Verlauf der schraubenartig oder spiralartig gewundenen Wendel (240) der Schneckenwelle (200) fortsetzt.

6. Schneckenwelle (200) zum Einsatz in einer Schneckenpresse (300), aufweisend einen sich entlang einer Längsachse (L) erstreckenden Schaft (230) mit einer schraubenartig oder spiralartig gewundene Wendel (240) **gekennzeichnet durch,** mindestens eine Wendelschraubenfördereinheit (100) nach mindestens einem der Ansprüche 1 bis 5, sowie mindestens ein zum lösbaren Befestigen des Befestigungselements (34) ausgebildetes Befestigungsmittel (212).

7. Schneckenwelle nach Anspruch 6, **dadurch gekennzeichnet, dass** das Befestigungsmittel (212) dazu ausgelegt ist mit dem Befestigungselement (34) der Wendelschraubenfördereinheit (100) derart zusammenzuwirken, dass die Wendelschraubenfördereinheit (100) lösbar am Befestigungsbereich (40) der Schneckenwelle (200) befestigbar ist.

8. Schneckenwelle (200) nach Anspruch 6 oder 7, **dadurch gekennzeichnet, dass** der Befestigungsbereich (40) der Schneckenwelle (200) derart geformt ist, dass eine radiale Bewegung des auf dem Befestigungsbereich (40) aufliegenden Grundkörpers (10) zumindest einschränkt wird.

9. Schneckenwelle (200) nach mindestens einem der Ansprüche 6 bis 8, **gekennzeichnet durch** mindestens zwei Befestigungsbereiche (40), die durch mindestens eine Ausnehmung oder mindestens eine Nut des ansonsten zylinderförmigen Wellenkörperelements (210) gebildet sind und radial sowie axial versetzt zueinander angeordnet sind.

10. Schneckenwelle (200) nach mindestens einem der Ansprüche 6 bis 9, **dadurch gekennzeichnet, dass** der Schaft (230) einen zylinderförmigen Wellenkörper sowie den Befestigungsbereich (40) aufweist, wobei der Befestigungsbereich (40) einem zum Stützen des Grundkörpers (10) der Wendelschraubenfördereinheit (100) ausgebildeten Stützbereich (210) des Schafts (230) zugeordnet ist und einen im Vergleich zum zylinderförmigen Wellenkörper geringeren Querschnittsdurchmesser aufweist.

11. Schneckenwelle (200) nach Anspruch 10, **dadurch gekennzeichnet, dass** der Stützbereich (210) exzentrisch, insbesondere an einem endnahen, beispielsweise an einem endständigen, Bereich des Schafts (230) angeordnet ist.

12. Schneckenwelle nach Anspruch 10 oder 11, **dadurch gekennzeichnet, dass** der Stützbereich (210) mindestens zwei nach mindestens einem der Ansprüche 1 bis 5 ausgebildete Wendelschraubenfördereinheiten (100) aufweist, wobei die Wendelschraubenfördereinheiten (100) derart angeordnet sind, dass die Wendelelemente (30) der Wendelschraubenfördereinheiten (100) den Verlauf der Wellenkörperwendel (240) des zylinderförmigen Wellenkörpers fortsetzen und mit diesen eine gemeinsame Wellenkörperwendeleinheit bilden.

13. Schneckenwelle nach mindestens einem der Ansprüche 10 bis 12, **dadurch gekennzeichnet, dass** die Schneckenwelle mindestens zwei Wendelschraubenfördereinheiten (100) aufweist, die (100) jeweils benachbart zueinander angeordnet sind und gemeinsam eine ganze, den Schaft (230) einmal vollständig umlaufende, Wendelumdrehung formen.

14. Schneckenpresse (300) zum Entwässern einer Suspension, insbesondere zum Entwässern von mittels anaerober Vergärung von organischem Material erhaltenem Digestat, aufweisend eine Presskammer, in welcher eine Schneckenwelle (200) mit einem schraubenartig gewundenen Wendelement um ihre Längsachse (L) rotierbar gelagert (250) ist, **dadurch gekennzeichnet, dass** die Schneckenwelle (200) nach mindestens einem der Ansprüche 6 bis 13 ausgebildet ist, wobei der Schaft (230) der Schneckenwelle (200) mindestens zwei Wendelschraubenfördereinheiten (100) aufweist, insbesondere von mindestens zwei Wendelschraubenfördereinheiten (100) ummantelt ist, und wobei die Wendelschraubenfördereinheiten (100) nach mindestens einem der Ansprüche 1 bis 5 ausgebildet sind.

15. Verfahren zum Bereitstellen einer zum Einsatz in einer Schneckenpresse (300) ausgebildeten Schneckenwelle (200) **dadurch gekennzeichnet, dass**
- eine, nach mindestens einem der Ansprüche 1 bis 5 ausgebildete, Wendelschraubenfördereinheit (100) an dem Befestigungsbereich (40) angeordnet wird und
- mittels dem dieser Wendelschraubenfördereinheit (100) zugeordneten Befestigungselement (34) lösbar an dem Befestigungsbereich (40) befestigt wird.

## Claims

1. Helical screw conveyor unit (100) configured as an exchangeable element for a helicoidally or spirally winding helix (240) of a worm shaft (200), having a main body (10) extending along a longitudinal axis (L), the main body (10) having two axial end regions (12, 14) and a lateral surface that connects the end regions (12, 14) to one another, at least one helicoidally winding helix element (30) being arranged on a top side (16) of the lateral surface and extending radially outward away from the top side (16),
**characterized in that**
- the top side (16) of the lateral surface is concavely curved in shell-like form about the longitudinal axis (L), and the main body (10) has a radial opening (20), the radial opening (20) being configured such that a fastening region (40) of a shank (230) of the worm shaft (200) can be led through the opening (20) in a direction perpendicular to the longitudinal axis (L) and can be arranged in a cavity formed by the main body (10),
- the helix element (30) has at most one half of one helix turn,
- the lateral surface of the main body (10) has an inner surface (18) configured for being seated on the fastening region (40), and
- the helical screw conveyor unit (100) has at least one fastening element (34) configured for detachably fastening the main body (10) to the fastening region (40).

2. Helical screw conveyor unit as claimed in claim 1, **characterized in that** the main body (10) runs in the shape of a semicircle or in the shape of a half-shell about the longitudinal axis (L), in particular is configured as a half-shell, and the helix element (30) has one half of one helix turn.

3. Helical screw conveyor unit as claimed in claim 1 or 2, **characterized in that** the inner surface (18) of the main body (10) is shaped such that, as a result of the inner surface (18) of the main body (10) being seated on the fastening region (40) of the worm shaft (200), the orientation of the helix element (30) of the helical screw conveyor unit (100) on the worm shaft (200) is defined, and radial mobility of the main body (10) on the worm shaft (200) is at least restricted.

4. Helical screw conveyor unit as claimed in claim 3, **characterized in that**, for the orientation of the helix element (30) on the worm shaft (200), the inner surface (18) of the lateral surface has a contour of complementary shape with respect to the fastening region (40) of the shank (230).

5. Helical screw conveyor unit as claimed in claim 2 or 3, **characterized in that** the helix element (30) is shaped such that, as a result of the inner surface (18) of the main body (10) being seated on the fastening region (40) of the worm shaft (200), the helix element (30) is at least substantially oriented so as to continue the course of the helicoidally or spirally winding helix (240) of the worm shaft (200).

6. Worm shaft (200) for use in a worm extruder (300), having a shank (230) which extends along a longitudinal axis (L) and which has a helicoidally or spirally winding helix (240), **characterized by** at least one helical screw conveyor unit (100) as claimed in at least one of claims 1 to 5, and at least one fastening means (212) configured for detachably fastening the fastening element (34).

7. Worm shaft as claimed in claim 6, **characterized in that** the fastening means (212) is configured to interact with the fastening element (34) of the helical screw conveyor unit (100) such that the helical screw conveyor unit (100) is detachably fastenable to the fastening region (40) of the worm shaft (200).

8. Worm shaft (200) as claimed in claim 6 or 7, **characterized in that** the fastening region (40) of the worm shaft (200) is shaped such that a radial movement of the main body (10) seated on the fastening region (40) is at least restricted.

9. Worm shaft (200) as claimed in at least one of claims 6 to 8, **characterized by** at least two fastening regions (40) which are formed by at least one recess or at least one groove of the otherwise cylindrical shaft body element (210) and which are arranged so as to be radially and axially offset with respect to one another.

10. Worm shaft (200) as claimed in at least one of claims 6 to 9, **characterized in that** the shank (230) has a cylindrical shaft body and the fastening region (40), the fastening region (40) being assigned to a support region (210), configured for supporting the main body (10) of the helical screw conveyor unit (100), of the shank (230) and having a cross-sectional diameter smaller than that of the cylindrical shaft body.

11. Worm shaft (200) as claimed in claim 10, **characterized in that** the support region (210) is arranged eccentrically, in particular on a near-terminal region, for example on a terminal region, of the shank (230).

12. Worm shaft as claimed in claim 10 or 11, **characterized in that** the support region (210) has at least two helical screw conveyor units (100) configured as claimed in at least one of claims 1 to 5, the helical screw conveyor units (100) being arranged such that the helical elements (30) of the helical screw conveyor units (100) continue the course of the shaft body helix (240) of the cylindrical shaft body and, with this, form a common shaft body helix unit.

13. Worm shaft as claimed in at least one of claims 10 to 12, **characterized in that** the worm shaft has at least two helical screw conveyor units (100) which are in each case arranged adjacent to one another and together form one full helix turn that fully encircles the shank (230) once.

14. Worm extruder (300) for dewatering a suspension, in particular for dewatering digestate obtained by anaerobic digestion of organic material, having an extruder chamber in which a worm shaft (200) having a helicoidally winding helix element is mounted (250) so as to be rotatable about its longitudinal axis (L), **characterized in that** the worm shaft (200) is configured as claimed in at least one of claims 6 to 13, the shank (230) of the worm shaft (200) having at least two helical screw conveyor units (100), in particular being encased by at least two helical screw conveyor units (100), and the helical screw conveyor units (100) being configured as claimed in at least one of claims 1 to 5.

15. Method for providing a worm shaft (200) configured for use in a worm extruder (300), **characterized in that**
- a helical screw conveyor unit (100) configured as claimed in at least one of claims 1 to 5 is arranged on the fastening region (40) and
- is detachably fastened to the fastening region (40) by means of the fastening element (34) assigned to said helical screw conveyor unit (100).

## Revendications

1. Unité de convoyeur à vis hélicoïdale (100) configurée comme élément interchangeable pour une hélice à enroulement hélicoïdal ou en spirale (240) d'un arbre à vis sans fin (200), ayant un corps principal (10) s'étendant le long d'un axe longitudinal (L), le corps principal (10) ayant deux régions d'extrémité axiale (12, 14) et une surface latérale qui relie les régions d'extrémité (12, 14) l'une à l'autre, au moins un élément d'hélice à enroulement hélicoïdal (30) étant disposé sur un côté supérieur (16) de la surface latérale et s'étendant radialement vers l'extérieur en s'éloignant du côté supérieur (16),
**caractérisé par le fait que**
- le côté supérieur (16) de la surface latérale est concave en forme de coquille autour de l'axe longitudinal (L), et le corps principal (10) présente une ouverture radiale (20), l'ouverture radiale (20) étant configurée de telle sorte qu'une région de fixation (40) d'une tige (230) de l'arbre à vis sans fin (200) peut être amenée à travers l'ouverture (20) dans une direction perpendiculaire à l'axe longitudinal (L) et peut être disposée dans une cavité formée par le corps principal (10),
- l'élément d'hélice (30) a au plus un demi-tour d'hélice,
- la surface latérale du corps principal (10) présente une surface intérieure (18) configurée pour reposer sur la région de fixation (40), et
- l'unité de convoyeur à vis hélicoïdale (100) comporte au moins un élément de fixation (34) configuré pour fixer de manière amovible le corps principal (10) à la région de fixation (40).

2. Unité de convoyeur à vis hélicoïdale selon la revendication 1, **caractérisé en ce que** le corps principal (10) s'étend en forme de demi-cercle ou de demi-coquille autour de l'axe longitudinal (L), en particulier est configuré comme une demi-coquille, et l'élément d'hélice (30) a une moitié d'un tour d'hélice.

3. Unité de convoyeur à vis hélicoïdale selon la revendication 1 ou 2, **caractérisé en ce que** la surface intérieure (18) du corps principal (10) est façonnée de telle sorte que, suite à l'assise de la surface intérieure (18) du corps principal (10) sur la région de fixation (40) de l'arbre à vis sans fin (200), l'orientation de l'élément d'hélice (30) du convoyeur à vis hélicoïdale (100) sur l'arbre à vis sans fin (200) est définie, et la mobilité radiale du corps principal (10) sur l'arbre à vis sans fin (200) est au moins restreinte.

4. Unité de convoyeur à vis hélicoïdale selon la revendication 3, **caractérisé en ce que**, pour l'orientation de l'élément d'hélice (30) sur l'arbre à vis sans fin (200), la surface intérieure (18) de la surface latérale présente un contour de forme complémentaire par rapport à la région de fixation (40) de la tige (230).

5. Unité de convoyeur à vis hélicoïdale selon la revendication 2 ou 3, **caractérisé en ce que** l'élément d'hélice (30) est formé de telle sorte que, suite à la mise en place de la surface intérieure (18) du corps principal (10) sur la région de fixation (40) de l'arbre à vis sans fin (200), l'élément d'hélice (30) est au moins sensiblement orienté de manière à poursuivre le cours de l'hélice (240) s'enroulant de manière hélicoïdale ou en spirale de l'arbre à vis sans fin (200).

6. Arbre à vis sans fin (200) destiné à être utilisé dans une extrudeuse à vis sans fin (300), comportant une tige (230) qui s'étend le long d'un axe longitudinal (L) et qui présente une hélice (240) à enroulement hélicoïdal ou en spirale, **caractérisé par** au moins une unité de convoyeur à vis hélicoïdale (100) selon l'une au moins des revendications 1 à 5, et au moins un moyen de fixation (212) configuré pour fixer de manière amovible l'élément de fixation (34).

7. Arbre à vis sans fin selon la revendication 6, **caractérisé en ce que** le moyen de fixation (212) est configuré pour interagir avec l'élément de fixation (34) de l'unité de convoyeur à vis hélicoïdale (100) de sorte que l'unité de convoyeur à vis hélicoïdale (100) peut être fixée de manière amovible à la région de fixation (40) de l'arbre à vis sans fin (200).

8. Arbre à vis sans fin (200) selon la revendication 6 ou 7, **caractérisé en ce que** la région de fixation (40) de l'arbre à vis sans fin (200) est formée de telle sorte qu'un mouvement radial du corps principal (10) assis sur la région de fixation (40) est au moins limité.

9. Arbre à vis sans fin (200) selon au moins l'une des revendications 6 à 8, **caractérisé par** au moins deux zones de fixation (40) qui sont formées par au moins un creux ou au moins une rainure de l'élément du corps de l'arbre (210), par ailleurs cylindrique, et qui sont disposées de manière à être décalées radialement et axialement l'une par rapport à l'autre.

10. Arbre à vis sans fin (200) selon l'une au moins des revendications 6 à 9, **caractérisé en ce que** la tige (230) présente un corps d'arbre cylindrique et la région de fixation (40), la région de fixation (40) étant affectée à une zone de support (210), configurée pour supporter le corps principal (10) de l'unité de convoyeur à vis hélicoïdale (100), de la tige (230) et présentant un diamètre de section transversale inférieur à celui du corps d'arbre cylindrique.

11. Arbre à vis sans fin (200) selon la revendication 10, **caractérisé en ce que** la zone de support (210) est disposée de manière excentrique, notamment sur une région proche de l'extrémité, par exemple sur une région terminale, de la tige (230).

12. Arbre à vis sans fin selon la revendication 10 ou 11, **caractérisé en ce que** la zone de support (210) comporte au moins deux unités de convoyeur à vis hélicoïdale (100) configurées selon au moins l'une des revendications 1 à 5, les unités de convoyeur à vis hélicoïdale (100) étant disposées de telle sorte que les éléments d'hélice (30) des unités de convoyeur à vis hélicoïdale (100) poursuivent le cours de l'hélice du corps d'arbre (240) du corps d'arbre cylindrique et forment ainsi une unité d'hélice du corps d'arbre commune.

13. Arbre à vis sans fin selon l'une au moins des revendications 10 à 12, **caractérisé en ce que** l'arbre à vis sans fin comporte au moins deux unités de convoyeur à vis hélicoïdale (100) qui sont dans chaque cas disposées de manière adjacente et forment ensemble un tour d'hélice complet qui encercle entièrement la tige (230) une fois.

14. Extrudeuse à vis sans fin (300) pour la déshydratation d'une suspension, en particulier pour la déshydratation d'un digestat obtenu par digestion anaérobie de matières organiques, comportant une chambre d'extrusion dans laquelle est monté (250) un arbre à vis sans fin (200) comportant un élément d'hélice à enroulement hélicoïdal, de manière à pouvoir tourner autour de son axe longitudinal (L), **caractérisée en ce que** l'arbre à vis sans fin (200) est configuré selon l'une au moins des revendications 6 à 13, la tige (230) de l'arbre à vis sans fin (200) comportant au moins deux unités de convoyeur à vis hélicoïdale (100), en particulier étant encastrée dans au moins deux unités de convoyeur à vis hélicoïdale (100), et les unités de convoyeur à vis hélicoïdale (100) étant configurées selon l'une au moins des revendications 1 à 5.

15. Procédé de fabrication d'un arbre à vis sans fin (200) configuré pour être utilisé dans une extrudeuse à vis sans fin (300), **caractérisé en ce que**
- une unité de convoyeur à vis hélicoïdale (100) configurée selon au moins l'une des revendications 1 à 5 est disposée sur la région de fixation (40) et
- est fixée de manière amovible à la région de fixation (40) au moyen de l'élément de fixation (34) attribué à ladite unité de convoyeur à vis hélicoïdale (100).
